## Europäisches Patentamt
## European Patent Office
## Office européen des brevets

(19)

(11) Publication number: **0 037 149**
**B1**

(12)

# EUROPEAN PATENT SPECIFICATION

(45) Date of publication of patent specification: **07.03.84**

(51) Int. Cl.³: **C 07 C 45/00, C 07 C 47/02**

(21) Application number: **81200329.1**

(22) Date of filing: **25.03.81**

(54) Method for the preparation of aldehydes.

(30) Priority: **29.03.80 NL 8001878**

(43) Date of publication of application:
**07.10.81 Bulletin 81/40**

(45) Publication of the grant of the patent:
**07.03.84 Bulletin 84/10**

(84) Designated Contracting States:
**BE CH DE FR GB IT LI NL**

(56) References cited:
**DE - A - 1 245 352**
**GB - A - 1 269 167**
**GB - A - 1 444 484**
**US - A - 1 764 962**

(73) Proprietor: **STAMICARBON B.V.**
**Postbus 10**
**NL-6160 MC Geleen (NL)**

(72) Inventor: **De Graaf, Theodorus Franciscus**
**Reinierstraat 2**
**NL-6191 SH Beek (L.) (NL)**
Inventor: **Delahaye, Hubertus Johannes**
**Spekhouwerstraat 63**
**NL-6367 TT Voerendaal (NL)**

(74) Representative: **Pinckaers, August René et al,**
**OCTROOIBUREAU DSM Postbus 9**
**NL-6160 MA Geleen (NL)**

Courier Press, Leamington Spa, England.

# Method for the preparation of aldehydes

The invention relates to a method for the preparation of aldehydes by dehydrogenation of monovalent primary alcohols.

In the British patent specification 1.444.484 a method is described for the dehydrogenation of alcohols in the gas phase by means of a copper-containing catalyst, in which process the dehydrogenation is effected in the presence of water vapour and small quantities of an indifferent gas in order to obtain a long useful service life of the catalyst, a very good selectivity and a high conversion.

The result of the use of water vapour in such a dehydrogenation process is that, in the upgrading of the gaseous reaction mixture obtained, by condensing it and separating the condensate by distillation, problems will often arise because of the formation of an azeotrope, with water, of the reaction product to be recovered and possibly starting product present. It is true that such an azeotropic mixture can be recirculated, but in many cases it means a substantial recirculation of the desired product.

In applying the method according to the said British patent specification for the dehydrogenation of monovalent primary alcohols, the length of life of the catalyst and the selectivity of the conversion into aldehyde are, moreover, less satisfactory.

A method has now been found for the dehydrogenation of monovalent primary alcohols, in which process no water vapour need be added to the starting product and a very long length of life of the catalyst, as well as a high degree of selectivity, can be reached.

The method according to the invention for the preparation of aldehydes by dehydrogenation of monovalent primary alcohols in the gas phase by means of a copper-containing catalyst is characterized in that first the dehydrogenation is effected in the presence of an indifferent gas and subsequently the catalyst used in this process is applied for effecting the dehydrogenation in the presence of a mixture of an indifferent gas and hydrogen.

'It is noted that in one of the 14 examples of GB—A—1 269 167 (example II) a process is disclosed for the preparation of ketones from isopropanol wherein hydrogen is not present in· the gaseous reaction mixture during an initial period of the concerning reaction. However, the general teaching of GB—A—1 269 167 is that the presence of hydrogen is preferred during the complete reaction period. Against this teaching, the process according to the invention comprises an initial period in which no hydrogen should be present to maintain a high degree of selectivity during the subsequent reaction period being carried out in the presence of hydrogen'.

In applying the method according to the invention various monovalent primary alcohols can be used as starting product, particularly monovalent primary alcohols with 2—20 carbon atoms such as, for instance, ethanol octanol-1, decanol-1, dodecanol-1, 2-hexenol-1, undec-10-enol-1, 2 methylundecanol-1, phenyl-ethanol and cinnamic alcohol.

As indifferent gas, the use of nitrogen is preferred, but other indifferent gases can in principle be used as well. The quantity of indifferent gas (separately and in the mixture with hydrogen) may vary within wide limits, for instance 2—100 moles indifferent gas per mole alcohol originally present. A very suitable result can be achieved in applying 5—40 moles indifferent gas per mole alcohol originally present.

The quantity of hydrogen may also vary within wide limits, for instance 0.5—50 moles hydrogen per mole alcohol originally present. Preferably, 1—20 moles hydrogen are used per mole of alcohol originally present.

The period of the dehydrogenation according to the invention using only indifferent gas is terminated when the catalyst has reached the desired level of selectivity, which can generally be realized in 2—24 hours. Then, by using the mixture of indifferent gas and hydrogen, the desired level of selectivity of the catalyst can be maintained for a long time, for instance some mouths.

In applying the method according to the invention, copper-containing dehydrogenation catalysts, known per se, such as copper with, as carrier, for instance magnesium oxide, zinc oxide, chromic oxide, aluminium oxide, silicon oxide, or a mixture of two or more of the said oxides, can be used as catalyst. If desired, oxides of alkali metals and/or earth alkali metals can be added to the catalyst as promotor.

For the purpose of effecting the dehydrogenation according to the invention temperatures of 200—450°C are generally suitable. Preferably a temperature of 225—350°C is applied. By very gradually raising the temperature within the desired limits, for instance a temperature increase corresponding with an average of one degree Centigrade in three days, the activity of the catalyst can be kept constant for a long time.

The space velocity is chosen, in the dehydrogenation process, for instance between 0.05 and 10 kg alcohol per litre catalyst (bulk volume). A lower or higher space velocity can be applied in principle, but this does not result in any advantage.

The gaseous reaction mixture obtained in applying the method according to the invention can be separated, by cooling, into a condensate, containing the desired product, and a gas mixture, which can be recirculated, if desired. If this gas mixture is recirculated, the quantity of hydrogen formed must, however, be removed in view of the formation of hydrogen in the dehydrogenation process. The condensate obtained in said cooling process can be separated by distillation into the aldehyde formed and, possibly, non-converted alcohol, which can be used again.

The aldehydes obtained in applying the method according to the invention can be used as raw material for the preparation of various valuable products, such as perfumes.

In the following examples the invention is further elucidated.

Example I

Through a vertical glass tube-shaped reactor (internal diameter 25 mm, height 40 cm), filled with 25 ml catalyst granules (Cu on MgO, 45% weight of Cu, diameter and length of the granules 3 and 5 mm respectively) a gaseous mixture of octanol-1 (16.5 g per hour) and nitrogen (20 moles per mole octanol) is passed, for 10 hours, over the catalyst from top to bottom at atmospheric pressure. By means of jacket heating the temperature in the reactor (measured about 1 mm above the catalyst) is kept at about 265°C. Subsequently, for a period of 4 weeks, a gaseous mixture of hydrogen, nitrogen and octanol-1 (16.5 g octanol per hour, 1 mole hydrogen and 9 moles nitrogen per mole octanol) is passed over the catalyst with very gradual increase in temperature (at the end of said period the temperature is 329°C), and the gaseous reaction mixture then obtained is removed via a receiving tank cooled at 12°C. The condensate thus formed in the receiving tank is periodically analysed gaschromatographically. This analysis shows that, 4 days after commencement of the passing over of hydrogen-containing gas mixture, the selectivity is 99% and the octanol conversion 58%. During the rest of the experiment the selectivity and the conversion continue to be 99 and 58% respectively.

The catalyst used in this example is prepared as known in the art by precipitation from a solution containing copper and magnesium salt, followed by filtration drying, granulation and reduction of the granules in the reactor with a hydrogen-nitrogen mixture at a temperature of 265°C.

Example II

Example I is repeated, however with application of a different molar ratio hydrogen:nitrogen:octanol, viz. 2.5:7.5:1. Four days after commencement of the passing across of hydrogen-containing gas mixture the selectivity is 99% and the conversion 45%. The experiment is ended after 12 weeks (at the end of this period the temperature is 313°C) without any decrease of selectivity and conversion.

Example III

Example II is repeated, however with application of a temperature of 275°C (instead of the temperature of 265°C as mentioned in Example I). The experiment is ended after 6 weeks (at the end of this period the temperature is 317°C). The selectivity is 99% and the conversion 55%.

Comparative Example A

Example II is repeated, however without the initial period (as described in example I) in which the octanol/nitrogen mixture is passed over for 10 hours. During an experiment of 6 weeks (at the end of this period the temperature is 290°C) the selectivity is only 30%. The conversion is 75%. This low selectivity is unacceptable in practice.

Comparative Example B

Example I is repeated. Instead of 1 mole hydrogen and 9 moles nitrogen, 10 moles nitrogen per mole octanol are now used. It is found that the conversion now shows a rapid decline, viz. about 10% a day (owing to temperature rise, this decline cannot be avoided while maintaining a proper selectivity), so that the experiment is ended after a few days.

Comparative Example C

Example I is repeated. Instead of 1 mole hydrogen and 9 moles nitrogen, 5 moles hydrogen per mole octanol are now used. Instead of a temperature of 265°C as in example I, a temperature of 305°C is applied to reach a good conversion (50%). It is found that a good conversion with a proper selectivity can be maintained for only a short time (a few days).

Example IV

Example I is repeated with decanol-1. First a nitrogen/decanol mixture (20 moles nitrogen per mole decanol, 16.6 g decanol per hour) is passed, for 10 hours, over the catalyst and subsequently hydrogen (2.5 moles hydrogen and 7.5 moles nitrogen per mole decanol) as well. The receiving tank is cooled at 30°C. After the hydrogen/nitrogen/decanol mixture has been passed over for about 4 days, a selectivity of 99% is reached, with a conversion of 40%, which values are maintained during the experiment, which lasts 6 weeks (at the end of the experiment the temperature is 289°C).

Example V

Example IV is repeated, with application, however, of a temperature of 275°C (instead of the temperature of 265°C as mentioned in example I). After the hydrogen-containing mixture has been passed over for about 4 days, the selectivity is 99% and the conversion 50%, which values are maintained during the experiment, which lasts 6 weeks (temperature at the end of the experiment 317°C).

**Claims**

1. Method for the preparation of aldehydes by dehydrogenation of monovalent primary alcohols in the gas phase by means of a copper-containing catalyst, characterized in that first the dehydrogenation is effected in the presence of an indifferent gas and subsequently the catalyst used in this process is applied in effecting the dehydrogenation in the presence of an indifferent gas and of hydrogen.

2. Method according to claim 1, characterized in that, per mole alcohol originally present, 5—40 moles indifferent gas are used.

3. Method according to claim 1 or 2, characterized in that, per mole alcohol originally present, 1—20 moles hydrogen are used.

. 4. Method according to any one of the claims 1—3, characterized in in that the dehydrogenation is effected at a temperature of 225—350°C.

5. Method according to any one of the claims 1—4, characterized in that, in the dehydrogenation process in the presence of the indifferent gas and of hydrogen, the temperature is increased gradually.

## Revendications

1. Procédé pour la préparation d'aldéhydes par déshydrogénation d'alcools primaires monovalents en phase gazeuse au moyen d'un catalyseur contenant du cuivre, caractérisé en ce que tout d'abord on effectue la déshydrogénation en présence d'un gaz inerte puis on emploie le catalyseur utilisé dans cette opération pour effectuer la déshydrogénation en présence d'un gaz inerte et d'hydrogène.

2. Procédé selon la revendication 1, caractérisé en ce que par mole d'alcool présente au départ, on emploie 5 à 40 moles de gaz inerte.

3. Procédé selon la revendication 1 ou 2, caractérisé en ce que par mole d'alcool présente au départ, on emploie 1 à 20 moles d'hydrogène.

4. Procédé selon l'une quelconque des revendications 1 à 3, caractérisé en ce qu'on effectue la déshydrogénation à une température de 225—350°C.

5. Procédé selon l'une quelconque des revendications 1 à 4, caractérisé en ce que dans l'opération de déshydrogénation en présence du gaz inerte et d'hydrogène, la température est élevée progressivement.

## Patentansprüche

1. Verfahren zur Herstellung von Aldehyden durch Dehydrierung von einwertigen Alkoholen in der gasförmigen Phase mit Hilfe eines Kupfer enthaltenden Katalysators, dadurch gekennzeichnet, dass zunächst die Dehydrierung in Anwesenheit eines indifferenten Gases durchgeführt wird und anschliessend der in diesem Prozess verwendete Katalysator bei der Dehydrierung in Anwesenheit eines indifferenten Gases und von Wasserstoff angewandt wird.

2. Verfahren nach Anspruch 1, dadurch gekennzeichnet, dass je ursprünglich vorhandenem Mol Alkohol 5—40 Mol indifferentes Gas verwendet werden.

3. Verfahren nach Anspruch 1 oder 2, dadurch gekennzeichnet, dass je ursprünglich vorhandenem Mol Alkohol 1—20 Mol Wasserstoff verwendet werden.

4. Verfahren nach einem der Ansprüche 1—3, dadurch gekennzeichnet, dass die Dehydrierung bei einer Temperatur von 225—350°C durchgeführt wird.

5. Verfahren nach einem der Ansprüche 1—4, dadurch gekennzeichnet, dass im Dehydrierprozess in Anwesenheit des indifferenten Gases und von Wasserstoff die Temperatur allmählich erhöht wird.